# EUROPÄISCHE PATENTSCHRIFT

(11) **EP 0 881 211 B1**
(45) Veröffentlichungstag und Bekanntmachung des Hinweises auf die Patenterteilung: **16.08.2000**
(21) Anmeldenummer: 98109538.3
(22) Anmeldetag: 26.05.1998
(51) Int. Cl.: C07C 213/10, C07C 213/02, C07C 217/08

(54) **Verfahren zur Herstellung von wasserfreiem 2-Amino-1-methoxypropan**
Process for the preparation of anhydrous 2-amino-1-methoxypropane
Procédé pour la préparation d'amino-2-méthoxy-1-propane anhydre

(30) Priorität: 30.05.1997 DE 19722700
(43) Veröffentlichungstag der Anmeldung: 02.12.1998
(73) Patentinhaber: BASF AKTIENGESELLSCHAFT, 67056 Ludwigshafen (DE)
(72) Erfinder: Simon, Joachim, Dr., 68161 Mannheim (DE); Henne, Andreas, Dr., 67433 Neustadt (DE); Lingk, Heinz, 67240 Bobenheim-Roxheim (DE)
(74) Vertreter: Isenbruck, Günter, Dr.

(56) Entgegenhaltungen:
- EP-A- 0 483 746
- EP-A- 0 508 280
- GB-A- 961 174
- US-A- 3 433 788

## Beschreibung

Die Erfindung betrifft ein Verfahren zur Herstellung von wasserfreiem 2-Amino-1-methoxypropan durch Abtrennung von Wasser aus wasserhaltigen Reaktionsgemischen, die durch Umsetzung von 1-Methoxy-2-propanol mit Ammoniak an einem Katalysator erhältlich sind. 2-Amino-1-methoxypropan ist ein wichtiges Zwischenprodukt für die Herstellung vieler chemischer Verbindungen.

Unterschiedliche Verfahren zur Herstellung von 2-Amino-1-methoxypropan durch Umsetzung von 1-Methoxy-2-propanol mit Ammoniak an Katalysatoren sind bekannt. Dabei werden jeweils wasserhaltige Reaktionsgemische erhalten, aus denen das Wasser entfernt werden muß, um wasserfreies 2-Amino-1-methoxypropan zu erhalten.

In der US 5,175,369 ist ein derartiges Verfahren beschrieben, bei dem 1-Methoxy-2-propanol und Ammoniak an einem Festbettkatalysator umgesetzt werden. Als Katalysator werden in der Regel Nickel- oder Kobaltkatalysatoren eingesetzt. Das erhaltene Reaktionsgemisch wird mit Diisopropylamin als Schleppmittel versetzt, und anschließend wird eine Destillation durchgeführt. Danach müssen Wasser und Schleppmittel getrennt werden, um das Schleppmittel in die Destillation zurückführen zu können.

In der US 5,074,967 ist ein Verfahren zur Herstellung von 2-Amino-1-methoxypropan beschrieben, bei dem 1-Methoxy-2-propanol mit Ammoniak am Nickelkatalysator umgesetzt wird. Das Reaktionsprodukt wird einer Reihe von Destillationsschritten unterworfen. Dabei wird zunächst bei gezielt eingestelltem, erhöhtem Druck destilliert, um ein Wasser/2-Amino-1-methoxy-propan-Azeotrop zu entfernen und ein wasserfreies Sumpfprodukt zu erhalten. Das abgetrennte Azeotrop wird sodann bei vermindertem Druck destilliert, wobei wiederum ein Azeotrop erhalten wird, das jedoch einen wesentlich geringeren Wasseranteil aufweist. Dieses Azeotrop wird in den Prozeß zurückgeführt. Bei diesem Verfahren werden Gesamtausbeuten von weniger als 65% erhalten.

Beide Verfahren sind durch den Einsatz eines Schleppmittels oder die Durchführung einer Vielzahl von Destillationen unter speziellen Bedingungen aufwendig und kostspielig in der Durchführung. Zudem sind die erreichten Ausbeuten gering.

Aufgabe der vorliegenden Erfindung ist die Bereitstellung eines Verfahrens zur Herstellung von wasserfreiem 2-Amino-1-methoxypropan aus 2-Amino-1-methoxypropan enthaltenden wasserhaltigen Reaktionsgemischen, das die Nachteile der bekannten Verfahren vermeidet und das gewünschte Produkt in einer hohen Gesamtausbeute liefert.

Die Aufgabe wird erfindungsgemäß gelöst durch ein Verfahren zur Herstellung von wasserfreiem 2-Amino-1-methoxypropan durch
(a) Versetzen eines 2-Amino-1-methoxypropan enthaltenden wasserhaltigen Reaktionsgemisches, erhältlich aus der Umsetzung von 1-Methoxy-2-propanol mit Ammoniak an einem Katalysator, mit Natronlauge unter Ausbildung einer Natronlauge enthaltenden wäßrigen Phase und einer 2-Amino-1-methoxypropan enthaltenden Phase,
(b) Abtrennung der 2-Amino-1-methoxypropan enthaltenden Phase von der wäßrigen Phase und
(c) Destillation der 2-Amino-1-methoxypropan enthaltenden Phase, wobei zuerst ein Azeotrop aus Wasser und 2-Amino-1-methoxypropan, das in Schritt (a) oder (b) zurückgeführt wird, und dann wasserfreies 2-Amino-1-methoxypropan gewonnen wird.

Zudem betrifft die Erfindung ein Verfahren zur Abtrennung von Wasser aus wasserhaltigen Reaktionsgemischen, erhältlich aus der Umsetzung von 1-Methoxy-2-propanol mit Ammoniak, durch
(a) Versetzen des Reaktionsgemisches mit Natronlauge unter Ausbildung einer Natronlauge enthaltenden wäßrigen Phase und einer 2-Amino-1-methoxypropan enthaltenden Phase,
(b) Abtrennung der 2-Amino-1-methoxypropan enthaltenden Phase von der wäßrigen Phase und gegebenenfalls
(c) Destillation der 2-Amino-1-methoxypropan enthaltenden Phase, wobei zuerst ein Azeotrop aus Wasser und 2-Amino-1-methoxypropan erhalten wird, das in Schritt (a) oder (b) zurückgeführt wird und dann wasserfreies 2-Amino-1-methoxypropan gewonnen wird.

Erfindungsgemäß wurde gefunden, daß Wasser aus 2-Amino-1-methoxypropan enthaltenden Reaktionsgemischen abgetrennt werden kann, wenn das Reaktionsgemisch mit Natronlauge versetzt wird, wobei sich ein zweiphasiges System ausbildet, und die so erhaltene 2-Amino-1-methoxypropan enthaltende Phase abgetrennt wird. In der Regel wird die abgetrennte Phase sodann einer Destillation unterworfen, um zu wasserfreiem 2-Amino-1-methoxypropan zu gelangen.

Vorzugsweise werden die vorstehenden Verfahrensschritte (a) und (b) wiederholt durchgeführt, wobei die jeweils in Schritt (b) erhaltene 2-Amino-1-methoxypropan enthaltende Phase im jeweils nachfolgenden Schritt (a) mit Natronlauge versetzt wird. Die Schritte (a) und (b) werden vorzugsweise so oft durchgeführt, daß die schließlich erhaltene 2-Amino-1-methoxypropan enthaltende Phase maximal 5, vorzugsweise maximal 3 Gew.-% Wasser, bezogen auf die Phase, enthält. Dies kann einer Anzahl von 1 bis 5 Wiederholungen entsprechen. In der Regel enthält die Phase 3 bis 5 Gew.-% Wasser, wenn die Schritte (a) und (b) bis zu 3 mal durchgeführt werden.

Besonders bevorzugt werden die Schritte (a) und (b) jedoch kontinuierlich durchgeführt. Dabei werden sie vorzugsweise in einer Extraktionskolonne, einem Zentrifugalextraktor und/oder einer Extraktorkaskade durchgeführt. Entsprechende Vorrichtungen sind bekannt.

Bei der kontinuierlichen Verfahrensführung leitet man dabei das wasserhaltige Reaktionsgemisch und Natronlauge im Gegenstrom über eine Extraktionskolonne, einen Zentrifugalextraktor und/oder eine Extraktorkaskade. Die Extraktorkaskade ist vorzugsweise zwei- bis fünfstufig. Als Extraktionskolonnen eignen sich beispielsweise Füllkörper-, Siebboden-, Kaskaden-, Pulsations-, Rotations- und Zentrifugalkolonnen. Der Mixer-Settler-Extraktor kann auch platzsparend als Turmextraktor oder Kastenextraktor ausgeführt sein.

Die eingesetzte Natronlauge ist vorzugsweise mindestens 40%ig, besonders bevorzugt 45 bis 55%ig. Auch nach dem kontinuierlichen Verfahren beträgt der Wassergehalt der schließlich erhaltenen 2-Amino-1-methoxypropan enthaltenden Phase maximal 5 Gew.-% Wasser.

Die aus der Extraktion der Schritte (a) und (b) erhaltene 2-Amino-1-methoxypropan enthaltende Phase wird anschließend einer Reindestillation unterworfen, wobei das restliche Wasser zunächst als Azeotrop mit 2-Amino-1-methoxypropan übergeht. Darauf folgt wasserfreies 2-Amino-1-methoxypropan. Das zunächst erhaltene Azeotrop wird wieder in Schritt (a) oder (b) zurückgeführt, vorzugsweise dem wasserhaltigen Reaktionsgemisch zugeschlagen. Die abgetrennte Natronlauge enthaltende wäßrige Phase wird aufkonzentriert und die Natronlauge wieder in das Verfahren zurückgeführt.

Das wasserhaltige Reaktionsgemisch enthält vorzugsweise 10 bis 25 Gew.-%, besonders bevorzugt 15 bis 20 Gew.-% Wasser, 70 bis 85, besonders bevorzugt 75 bis 83 Gew.-% 2-Amino-1-methoxypropan, 0,2 bis 5, vorzugsweise 1 bis 4 Gew.-% 1-Methoxy-2-propanol und 0 bis 2, vorzugsweise 0,1 bis 1 Gew.-% Nebenprodukte, wobei die Gesamtmenge der Inhaltsstoffe 100 Gew.-% ergibt. Die Umsetzung von 1-Methoxy-2-propanol zu 2-Amino-1-methoxypropan erfolgt vorzugsweise an einem Katalysator, wie er in EP-A-0 394 842 oder EP-A-0 696 572 beschrieben ist. Vorzugsweise wird ein Katalysator aus 40 bis 60 Gew.-% NiO, 20 bis 40 Gew.-% ZrO₂, 10 bis 30 Gew.-% CuO und 0 bis 2 Gew.-% MoO₃ eingesetzt. Die Umsetzung erfolgt dabei nach dem in EP-A-0 696 572 beschriebenen Verfahren unter den dort angegebenen Bedingungen. Vorzugsweise liegt der Druck im Bereich von 21 bis 300 bar und die Temperatur im Bereich von 170 bis 220°C. Bevorzugt ist eine kontinuierliche Umsetzung in einem Festbettreaktor, der insbesondere in Rieselfahrweise betrieben wird. Der Umsatz, bezogen auf 1-Methoxy-2-propanol, beträgt dabei bis zu 99% bei einer Selektivität für 2-Amino-1-methoxypropan von bis zu 99,5%. Die Ausbeute der nachfolgenden Destillation beträgt beispielsweise 87%. Dies führt zu einer Gesamtausbeute von etwa 86%, wodurch das erfindungsgemäße Verfahren wesentlich bessere Ausbeuten liefert als das Verfahren gemäß US 5,074,967. Durch den hohen Umsatz wird die Aufarbeitung zum wasserfreien 2-Amino-1-methoxypropan erleichtert, da wenig Ausgangsstoffe abgetrennt werden müssen.

Nachstehend wird die Erfindung anhand von Beispielen näher erläutert.

### Beispiele

### 1. Herstellung des Katalysators

Der Katalysator wurde gemäß Beispiel 1a) aus EP-A-0 394 842 hergestellt. Dazu wurde eine wäßrige Lösung aus Nickelnitrat, Kupfernitrat und Zirkonacetat, die 4,48% NiO, 1,52% CuO und 2,82% ZrO₂ enthielt, gleichzeitig in einem Rührgefäß in einem konstanten Strom mit einer 20%igen wäßrigen Natriumcarbonatlösung bei einer Temperatur von 70°C so gefällt, daß der mit einer Glaselektrode gemessene pH-Wert von 7,0 konstant blieb. Die erhaltene Suspension wurde filtriert und der Filterkuchen mit vollentsalztem Wasser so lange gewaschen, bis die elektrische Leitfähigkeit des Filtrats rund 20 µS betrug. Dann wurde in den noch feuchten Filterkuchen so viel Ammoniumheptamolybdat eingearbeitet, daß das nachfolgend angegebene Oxidgemisch erhalten wurde. Danach wurde der Filterkuchen bei einer Temperatur von 150°C in einem Trockenschrank oder einem Sprühtrockner getrocknet. Das auf diese Weise erhaltene Hydroxidcarbonatgemisch wurde nun bei einer Temperatur von 500°C über einen Zeitraum von vier Stunden getempert. Der so erhaltene Katalysator hatte die Zusammensetzung: 50% NiO, 17% CuO, 1,5% MoO₃ und 31,5% ZrO₂. Das Katalysatorpulver wurde mit 3% Graphit vermischt und zu 6 x 3 mm Tabletten verformt. Die Tabletten hatten eine Porosität (gemessen über die Wasseraufnahme) von 0,20 ml/g und eine Härte von 3500 N/cm².

### 2. Herstellung des Rohprodukts

Die Reaktion wird in einem kontinuierlich betriebenen Hochdruckreaktor vorgenommen. Der elektrisch beheizte Reaktor besteht aus V₄A-Stahl und hat bei einer Länge von 300 cm einen Innendurchmesser von 3 cm. Während der Umsetzung, die in Rieselfahrweise durchgeführt wird, werden außer Ammoniak und 1-Methoxy-2-propanol noch 300 Nl/h Wasserstoff durch den Reaktor geleitet. Hinter dem Reaktor wird das Produkt in üblicher Weise kondensiert, entspannt und ausgetragen.

Über eine Schüttung von 500 ml des wie vorstehend unter Ziffer 1 hergestellten Katalysators werden bei einem Gesamtdruck von 200 bar und einer Temperatur von 200°C stündlich 700 g Ammoniak und 140 g 1-Methoxy-2-propanol geleitet. Man erhält ein Produkt mit folgender Zusammensetzung (Angaben in GC-Flächen-%, wasser- und ammoniakfrei gerechnet):
96,8% 2-Amino-1-methoxypropan
2,6% unumgesetztes 1-Methoxy-2-propanol
0,6% Nebenprodukte
Der Umsatz an 1-Methoxy-2-propanol beträgt 97,4% und die Selektivität für 2-Amino-1-methoxypropan 99,4%.
Das Rohprodukt enthält 17 Gew.-% Wasser.

### 3. Herstellung von reinem, wasserfreien 2-Amino-1-methoxypropan

3,5 kg eines Rohprodukts mit der oben unter Ziffer 2 angegebenen Zusammensetzung werden zur Entwässerung zunächst mit 0,6 kg 50%iger Natronlauge versetzt und gerührt. Anschließend läßt man absitzen und trennt die obere, organische Phase ab. Man wiederholt diesen Vorgang noch zweimal, bis der Wassergehalt in der organischen Phase noch 3 bis 5% beträgt. Anschließend wird die organische Phase bei Normaldruck über eine Füllkörperkolonne mit etwa 20 theoretischen Böden rektifiziert. Die mit einem verspiegelten Vakuummantel versehene, 1 m lange Kolonne hat einen Innendurchmesser von 50 mm und ist mit 5 mm großen V₂A-Netzringen gefüllt. Die Durchführung der Destillation ist in der folgenden Tabelle zusammengefaßt.

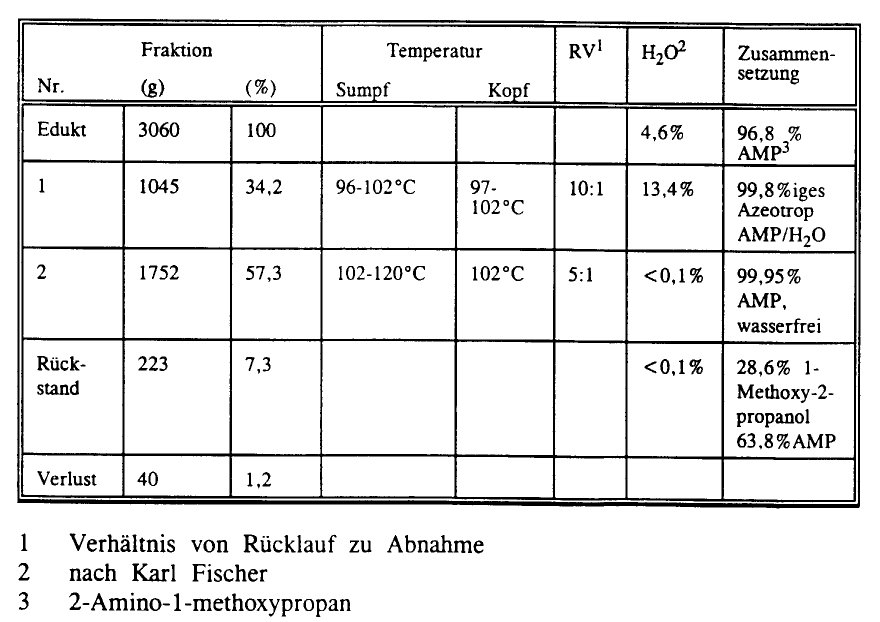

Die hauptsächlich Wasser und 2-Amino-1-methoxypropan enthaltende Fraktion 1 wird mit Rohprodukt gemischt, das wiederum mit Natronlauge entwässert und aufdestilliert wird. Durch diese Rückführung erhält man insgesamt eine Destillationsausbeute von 87%, bezogen auf den Gehalt an 2-Amino-1-methoxypropan im Rohprodukt. Die Gesamtbeute an reinem, wasserfreiem 2-Amino-1-methoxypropan über Synthese und Reinigung ergibt sich damit zu 86% der Theorie.

### Vergleichsbeispiel

Ein gemäß US 5,074,967 durch hydrierende Aminierung von 1-Methoxy-2-propanol mit Ammoniak hergestellter Rohaustrag hat folgende Zusammensetzung (GC-Flächen %, wasser- und ammoniakfrei gerechnet):
67% 2-Amino-1-methoxypropan
30% 1-Methoxy-2-propanol
3 % Nebenprodukte

Der Umsatz an 1-Methoxy-2-propanol beträgt 70% und die Selektivität für 2-Amino-1-methoxypropan 96%. Das Rohprodukt enthält 12 Gew.-% Wasser. Die in der US 5,074,967 beschriebene mehrstufige Druckdestillation ergibt in der Nacharbeitung eine Destillationsausbeute von 90%. Damit beläuft sich die Gesamtbeute an wasserfreiem 2-Amino-1-methoxypropan in diesem Verfahren auf 61% der Theorie.

## Patentansprüche

1. Verfahren zur Herstellung von wasserfreiem 2-Amino-1-methoxypropan durch
(a) Versetzen eines 2-Amino-1-methoxypropan enthaltenden wasserhaltigen Reaktionsgemisches, erhältlich aus der Umsetzung von 1-Methoxy-2-propanol mit Ammoniak an einem Katalysator, mit Natronlauge unter Ausbildung einer Natronlauge enthaltenden wäßrigen Phase und einer 2-Amino-1-methoxypropan enthaltenden Phase,
(b) Abtrennung der 2-Amino-1-methoxypropan enthaltenden Phase von der wäßrigen Phase und
(c) Destillation der 2-Amino-1-methoxypropan enthaltenden Phase, wobei zuerst ein Azeotrop aus Wasser und 2-Amino-1-methoxypropan, das in Schritt (a) oder (b) zurückgeführt wird, und dann wasserfreies 2-Amino-1-methoxypropan gewonnen wird.

2. Verfahren zur Abtrennung von Wasser aus wasserhaltigen Reaktionsgemischen, erhältlich aus der Umsetzung von 1-Methoxy-2-propanol mit Ammoniak, durch
(a) Versetzen des Reaktionsgemisches mit Natronlauge unter Ausbildung einer Natronlauge enthaltenden wäßrigen Phase und einer 2-Amino-1-methoxypropan enthaltenden Phase,
(b) Abtrennung der 2-Amino-1-methoxypropan enthaltenden Phase von der wäßrigen Phase und gegebenenfalls
(c) Destillation der 2-Amino-1-methoxypropan enthaltenden Phase, wobei zuerst ein Azeotrop aus Wasser und 2-Amino-1-methoxypropan erhalten wird, das in Schritt (a) oder (b) zurückgeführt wird und dann wasserfreies 2-Amino-1-methoxypropan gewonnen wird.

3. Verfahren nach Anspruch 1 oder 2, dadurch gekennzeichnet, daß die Schritte (a) und (b) wiederholt durchgeführt werden, wobei die jeweils in Schritt (b) erhaltene 2-Amino-1-methoxypropan enthaltende Phase im jeweils nachfolgenden Schritt (a) mit Natronlauge versetzt wird.

4. Verfahren nach Anspruch 1 oder 2, dadurch gekennzeichnet, daß die Schritte (a) und (b) kontinuierlich durchgeführt werden.

5. Verfahren nach Anspruch 4, dadurch gekennzeichnet, daß die Schritte (a) und (b) in einer Extraktionskolonne, einem Zentrifugalextraktor und/oder einer Extraktorkaskade durchgeführt werden.

6. Verfahren nach einem der Ansprüche 3 bis 5, dadurch gekennzeichnet, daß die in Schritt (c) eingesetzte 2-Amino-1-methoxypropan enthaltende Phase maximal 5 Gew.-% Wasser, bezogen auf die eingesetzte Phase, enthält.

7. Verfahren nach einem der Ansprüche 1 bis 6, dadurch gekennzeichnet, daß das wasserhaltige Reaktionsgemisch 10 bis 25 Gew.-% Wasser, 70 bis 85 Gew.-% 2-Amino-1-methoxypropan, 0,2 bis 5 Gew.-% 1-Methoxy-2-propanol und 0 bis 2 Gew.-% Nebenprodukte enthält, wobei die Gesamtmenge der Inhaltsstoffe 100 Gew.-% ergibt.

8. Verfahren nach einem der Ansprüche 1 bis 7, dadurch gekennzeichnet, daß das wasserhaltige Reaktionsgemisch durch Umsetzung von 1-Methoxy-2-propanol mit Ammoniak hergestellt wird, wobei die Umsetzung eines oder mehrere der nachstehenden Merkmale aufweist:
- Druck im Bereich von 21 bis 300 bar;
- Temperatur im Bereich von 170 bis 220°C;
- Kontinuierliche Umsetzung an einem Festbettkatalysator;
- Umsetzung in Rieselfahrweise;
- Katalysator aus 40 bis 60 Gew.-% NiO, 20 bis 40 Gew.-% ZrO₂, 10 bis 30 Gew.-% CuO und 0 bis 2 Gew.-% MoO₃, wobei die Gesamtmenge 100 Gew.-% ergibt.

9. Verfahren nach einem der Ansprüche 1 bis 8, dadurch gekennzeichnet, daß die eingesetzte Natronlauge mindestens 40%ig ist.

## Claims

1. A process for preparing anhydrous 2-amino-1-methoxypropane by
(a) admixing a 2-amino-1-methoxypropane-containing water-containing reaction mixture, obtainable from the reaction of 1-methoxy-2-propanol with ammonia on a catalyst, with sodium hydroxide solution, forming an aqueous phase containing sodium hydroxide solution and a 2-amino-1-methoxypropane-containing phase,
(b) separating off the 2-amino-1-methoxypropane-containing phase from the aqueous phase and
(c) distilling the 2-amino-1-methoxypropane-containing phase, an azeotrope of water and 2-amino-1-methoxypropane, which is recycled to step (a) or (b), first being produced and anhydrous 2-amino-1-methoxypropane then being produced.

2. A process for separating off water from water-containing reaction mixtures, obtainable from the reaction of 1-methoxy-2-propanol with ammonia, by
(a) admixing the reaction mixture with sodium hydroxide solution, forming an aqueous phase containing sodium hydroxide solution and a 2-amino-1-methoxypropane-containing phase,
(b) separating off the 2-amino-1-methoxypropane-containing phase from the aqueous phase, with or without
(c) distilling the 2-amino-1-methoxypropane-containing phase, an azeotrope of water and 2-amino-1-methoxypropane, which is recycled to step (a) or (b), first being obtained and anhydrous 2-amino-1-methoxypropane then being produced.

3. A process as claimed in claim 1 or 2, wherein the steps (a) and (b) are carried out repeatedly, the 2-amino-1-methoxypropane-containing phase obtained in each step (b) being admixed with sodium hydroxide solution in each subsequent step (a).

4. A process as claimed in claim 1 or 2, wherein the steps (a) and (b) are carried out continuously.

5. A process as claimed in claim 4, wherein the steps (a) and (b) are carried out in an extraction column, a centrifugal extractor and/or an extractor cascade.

6. A process as claimed in one of claims 3 to 5, wherein the 2-amino-1-methoxypropane-containing phase used in step (c) contains at most 5% by weight of water, based on the phase used.

7. A process as claimed in one of claims 1 to 6, wherein the water-containing reaction mixture contains from 10 to 25% by weight of water, from 70 to 85% by weight of 2-amino-1-methoxypropane, from 0.2 to 5% by weight of 1-methoxy-2-propanol and from 0 to 2% by weight of byproducts, the total of the constituents being 100% by weight.

8. A process as claimed in one of claims 1 to 7, wherein the water-containing reaction mixture is prepared by reacting 1-methoxy-2-propanol with ammonia, the reaction having one or more of the features below:
- pressure in the range from 21 to 300 bar;
- temperature in the range from 170 to 220°C;
- continuous reaction on a fixed-bed catalyst;
- reaction in the trickle mode;
- catalyst of from 40 to 60% by weight of NiO, from 20 to 40% by weight of ZrO₂, from 10 to 30% by weight of CuO and from 0 to 2% by weight of MoO₃, the total amount being 100% by weight.

9. A process as claimed in one of claims 1 to 8, wherein the sodium hydroxide solution used is at least 40% strength.

## Revendications

1. Procédé de préparation de 2-amino-1-méthoxypropane anhydre, par
(a) addition, à un mélange réactionnel aqueux contenant du 2-amino-1-méthoxypropane et pouvant être obtenu par la réaction du 1-méthoxy-2-propanol avec de l'ammoniac sur un catalyseur, d'une solution aqueuse d'hydroxyde de sodium, avec formation d'une phase aqueuse contenant de l'hydroxyde de sodium et fd'une phase contenant du 2-amino-1-méthoxypropane,
(b) séparation, d'avec la phase aqueuse, de la phase contenant le 2-amino-1-méthoxypropane, et
(c) distillation de la phase contenant le 2-amino-1-méthoxypropane, au cours de laquelle on obtient d'abord un azéotrope d'eau et de 2-amino-1-méthoxypropane, qui est renvoyé dans l'étape (a) ou (b), puis du 2-amino-1-méthoxypropane anhydre.

2. Procédé de séparation de l'eau à partir de mélanges réactionnels aqueux, pouvant être obtenus par réaction de 1-méthoxy-2-propanol avec de l'ammoniac, par
(a) addition, au mélange réactionnel, d'une solution aqueuse d'hydroxyde de sodium, avec formation d'une phase aqueuse contenant de l'hydroxyde de sodium et d'une phase contenant du 2-amino-1-méthoxypropane,
(b) séparation, d'avec la phase aqueuse, de la phase contenant le 2-amino-1-méthoxypropane, et éventuellement
(c) distillation de la phase contenant le 2-amino-1-méthoxypropane, au cours de laquelle on obtient d'abord un azéotrope d'eau et de 2-amino-1-méthoxypropane, qui est renvoyé dans l'étape (a) ou (b), puis on obtient le 2-amino-1-méthoxypropane anhydre.

3. Procédé selon la revendication 1 ou 2, caractérisé en ce que les étapes (a) et (b) sont mises en oeuvre d'une manière répétitive, la phase contenant le 2-amino-1-méthoxypropane et obtenue dans chaque étape (b) étant, au cours de chaque étape (a) suivante, additionnée d'hydroxyde de sodium.

4. Procédé selon la revendication 1 ou 2, caractérisé en ce que les étapes (a) et (b) sont mises en oeuvre en continu.

5. Procédé selon la revendication 4, caractérisé en ce que les étapes (a) et (b) sont mises en oeuvre dans une colonne d'extraction, un extracteur centrifuge et/ou une cascade d'extracteurs.

6. Procédé selon l'une des revendications 3 à 5, caractérisé en ce que la phase contenant le 2-amino-1-méthoxypropane et utilisée dans l'étape (c) contient un maximum de 5 % en poids d'eau par rapport à la phase utilisée.

7. Procédé selon l'une des revendications 1 à 6, caractérisé en ce que le mélange réactionnel aqueux contient de 10 à 25 % en poids d'eau, de 70 à 85 % en poids de 2-amino-1-méthoxypropane, de 0,2 à 5 % en poids de 1-méthoxy-2-propanol et de 0 à 2 % en poids de sous-produits, la quantité totale des constituants étant de 100 % en poids.

8. Procédé selon l'une des revendications 1 à 7, caractérisé en ce que le mélange réactionnel aqueux est préparé par réaction de 1-méthoxy-2-propanol avec de l'ammoniac, la réaction présentant une ou plusieurs des caractéristiques suivantes :
- pression comprise entre 21 et 300 bar ;
- température comprise entre 170 et 220°C ;
- réaction continue sur un catalyseur à lit fixe ;
- réaction par le procédé par ruissellement ;
- catalyseur constitué de 40 à 60 % en poids de NiO, de 20 à 40 % en poids de ZrO₂, de 10 à 30 % en poids de CuO et de 0 à 2 % en poids de MoO₃, la quantité totale étant de 100 % en poids.

9. Procédé selon l'une des revendications 1 à 8, caractérisé en ce que la solution aqueuse d'hydroxyde de sodium utilisée est à au moins 40 %.
